# EUROPEAN PATENT SPECIFICATION

(11) **EP 1 651 624 B1**
(45) Date of publication and mention of the grant of the patent: **11.03.2015**
(21) Application number: 04779726.1
(22) Date of filing: 30.07.2004
(51) Int. Cl.: C07D 277/56

(54) **PROCESS FOR THE PREPARATION OF SUBSTITUTED THIAZOLES**
VERFAHREN ZUR HERSTELLUNG SUBSTITUIERTER THIAZOLE
PROCÉDÉ DE PRÉPARATION DES THIAZOLES SUBSTITUÉS

(30) Priority: 30.07.2003 US 630043
(43) Date of publication of application: 03.05.2006
(60) Divisional application: 11170183.5
(73) Proprietor: AbbVie Inc., North Chicago, IL 60064 (US)
(72) Inventor: ROBBINS, Timothy, A., Gurnee, Illinois 60031 (US); ZHU, Helen, South Bend, Indiana 46601 (US); SHAO, Jun, Mason, Ohio 45040 (US)
(74) Representative: Modiano, Micaela Nadia
(86) International application number: PCT/US2004/024758
(87) International publication number: WO 2005/012273

(56) References cited:
- EP-A- 0 097 323
- EP-A- 0 480 281
- WO-A-01/40207
- JP-A- 2002 053 567
- US-A- 3 518 279
- US-A- 3 852 287
- DATABASE BEILSTEIN BEILSTEIN INSTITUTE FOR ORGANIC CHEMISTRY, FRANKFURT-MAIN, DE; XP002303595 Database accession no. 6076059 & REISSERT; GRUBE: CHEM. BER., vol. 42, 1909, page 3712,
- RAPAPORT E ET AL: "CHEMILUMINESCENCE OF LINEAR HYDRAZIDES" JOURNAL OF THE AMERICAN CHEMICAL SOCIETY, AMERICAN CHEMICAL SOCIETY, WASHINGTON, DC, US, vol. 94, no. 9, 19 April 1972 (1972-04-19), pages 3153-3159, XP000608768 ISSN: 0002-7863
- HASEGAWA T: "A FACILE ONE-POT SYNTHESIS OF 4-ALKOXY-1,3-BENZENEDICARBONITRILE" HETEROCYCLES, ELSEVIER SCIENCE PUBLISHERS B.V. AMSTERDAM, NL, vol. 47, no. 2, 1998, pages 857-864, XP001056564 ISSN: 0385-5414
- PATENT ABSTRACTS OF JAPAN vol. 1999, no. 08, 30 June 1999 (1999-06-30) & JP 11 060552 A (TEIJIN LTD), 2 March 1999 (1999-03-02)
- CSAVASSY G ET AL: "THIAZOLVERBINDUNGEN, I SYNTHESE UND UMSETZUNG VON 2-ARYL-5-DIAZOACETYL-4-METHYL-THIAZOLEN THIAZOLE COMPOUNDS, I. - SYNTHESIS AND REACTIONS OF 2-ARYL-5-DIAZOACETYL-4-METHYLTHIAZOLES" JUSTUS LIEBIGS ANNALEN DER CHEMIE, VERLAG CHEMIE GMBH. WEINHEIM, DE, vol. 8, 1974, pages 1195-1205, XP001026825 ISSN: 0075-4617
- DATABASE CA [Online] CHEMICAL ABSTRACTS SERVICE, COLUMBUS, OHIO, US; ISTANBULLU, ISMAIL ET AL: "Studies of 2-(2-hydroxyphenyl)thiazole derivatives" XP002302864 retrieved from STN Database accession no. 1989:57560 & HACETTEPE UNIVERSITESI ECZACILIK FAKULTESI DERGISI , 6(1), 21-8 CODEN: HUEDEE; ISSN: 1300-0608, 1986,

## Description

### FIELD OF THE INVENTION

### BACKGROUND OF THE INVENTION

The compound ethyl 2-(4-hydroxyphenyl)-4-methyl-1,3-thiazole-5-carboxylate, also known as TEI-6720, is useful for treatment of gout and hyperuricemia. This compound belongs to a class of substituted thiazoles that inhibit xanthine oxidase and thus block uric acid production.

The synthesis of TEI-6720 involves two steps. In the first step, an aryl nitrile is converted to a thioamide. In the second step the thioamide is converted to a thiazole. Because of the therapeutic usefulness of TEI-6720 there is sustained interest in improving the synthesis of substituted thiazoles in general and TEI-6720 in particular.

US Patent No. 3,852,287 discloses the preparation of thionamides by treating the corresponding aryl and heteroaryl nitriles with hydrogen sulfide gas, in the presence of a basic catalyst. Ethanol as solvent is taught. The preparation is carried out by loading an autoclave, which is heated to a temperature of 80°-125°C and within which a pressure of 689476-1378952 Pa (100-200 psi) will develop. US Patent No. 3,852,287 also teaches formation of a thiazole from the reaction of thionacetamide with chloroacetoacetanilide in acetonitrile at a temperature of 65°-70°C.

### SUMMARY OF THE INVENTION

The scope of the present invention is defined by the appended claims. Accordingly, this invention is directed to a process for the preparation of a compound of formula (IV) 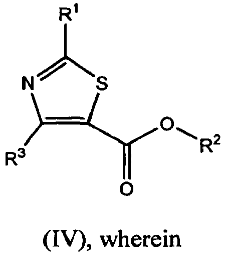
R¹ is selected from the group consisting of heteroaryl, phenyl, or phenyl substituted with one, two, three, or four substituents independently selected from the group consisting of C₁-C₆-alkyl, C₂-C₆-alkenyl, C₂-C₆-alkynyl, -OH, -F, -Cl, -Br, -I, -NH₂ and -NO₂;
R² is selected from the group consisting of hydrogen, C₁-C₆-alkyl, C₂-C₆-alkenyl, and C₂-C₆-alkynyl; and
R³ is selected from the group consisting of hydrogen, C₁-C₆-alkyl, C₂-C₆-alkenyl, and C₂-C₆-alkynyl;
the process comprising the steps of:
   (a) reacting a compound having formula (II)

      R¹-C≡N (II),

      with a base and H₂S to provide a compound of formula (I)

      R¹-(C=S)-NH₂ (I),

      wherein the base is a compound of formula (III)

      (M)⁺(YH)⁻ (III),

      wherein
      M is sodium, potassium, lithium, or -NH₄; and
      Y is oxygen or sulfur;
      wherein step (a) is conducted under a pressure of 413685.4 Pa (60 psi) at a temperature of 70 °C in ethanol;
      and
   (b) reacting the product of step (a) with a compound having formula (V) 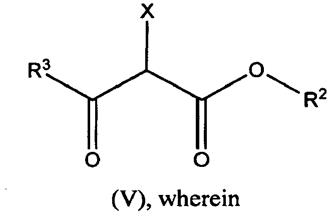
X is selected from the group consisting of -Cl, -Br, -I, and -F;
wherein step (b) is conducted at a temperature of about 80 °C in ethanol;
wherein the process is conducted as a continuous process.

In one embodiment, the process is for the preparation of ethyl-2-(4-hydroxyphenyl)-4-methyl-1,3-thiazole-5-carboxylate.

In a further embodiment, the base is sodium hydroxide.

### DETAILED DESCRIPTION OF THE INVENTION

The invention discloses a synthesis that eliminates the need for a catalyst, yet decreases processing time and increases the product yield. Overall, this invention allows large-scale synthesis and a commercially feasible process for making TEI-6720.

### DEFINITION OF TERMS

As used throughout this specification and the appended claims, the following terms have the following meanings:
All of the processes of the instant invention can be conducted as continuous processes. The term "continuous process, " as used herein, represents steps conducted without isolation of the intermediates.

The term "alkenyl" as used herein, means a straight or branched chain hydrocarbon containing from 2 to 6 carbons and containing at least one carbon-carbon double bond formed by the removal of two hydrogens. Representative examples of alkenyl include, but are not limited to, ethenyl, 2-propenyl, 2-methyl-2-propenyl, 3-butenyl, 4-pentenyl, and 5-hexenyl.

The term "alkyl" as used herein, means a straight or branched chain hydrocarbon containing from 1 to 6 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, and n-hexyl.

The term "alkynyl" as used herein, means a straight or branched chain hydrocarbon group containing from 2 to 6 carbon atoms and containing at least one carbon-carbon triple bond. Representative examples of alkynyl include, but are not limited, to acetylenyl, 1-propynyl, 2- propynyl, 3-butynyl, 2-pentynyl, and 1-butynyl.

The term "heteroaryl, " as used herein, refers to an aromatic five-or six-membered ring wherein 1, 2, 3, or 4 heteroatoms are independently selected from N, O, or S. The five membered rings have two double bonds and the six membered rings have three double bonds. The heteroaryl groups are connected to the parent molecular moiety through a carbon or nitrogen atom. The term "heteroaryl" also includes bicyclic systems where a heteroaryl ring is fused to a phenyl group, a monocyclic cycloalkyl group, as defined herein, a heterocycle group, as defined herein, or an additional heteroaryl group; and tricyclic systems where a bicyclic system is fused to a phenyl group, a monocyclic cycloalkyl group, as defined herein, a heterocycle group, as defined herein, or an additional heteroaryl group. Representative examples of heteroaryl include, but are not limited to, benzothienyl, benzoxadiazolyl, cinnolinyl, dibenzofuranyl, furopyridinyl, furyl, imidazolyl, indazolyl, indolyl, isoxazolyl, isoquinolinyl, isothiazolyl, naphthyridinyl, oxadiazolyl, oxazolyl, pyridinyl, pyridazinyl, pyrimidinyl, pyrazinyl, pyrazolyl, pyrrolyl, quinolinyl, tetrazolyl, thiadiazolyl, thiazolyl, thienopyridinyl, thienyl, triazolyl, and triazinyl.

The term "base, " as used herein, is a compound of formula (III). Examples of bases include hydroxides such as lithium hydroxide, sodium hydroxide, and potassium hydroxide. The base chosen for a particular conversion depends on the nature of the starting materials, the solvent or solvents in which the reaction is conducted, and the temperature at which the reaction is conducted.

The term "solvent, " as used herein, refers to the dispersing medium of a solution, which is ethanol in the process of the invention.

Percentages such as % yield were obtained by HPLC analyses of starting materials and products. Values were calculated from the peak area.

### Abbreviations

HPLC for high pressure liquid chromatography;

### Synthetic Methods

The methods of this invention as disclosed above will be better understood in connection with the following synthetic scheme. It will be readily apparent to one of ordinary skill in the art that the compounds (IV) can be prepared by substitution of the appropriate reactants and agents in the synthesis shown below.

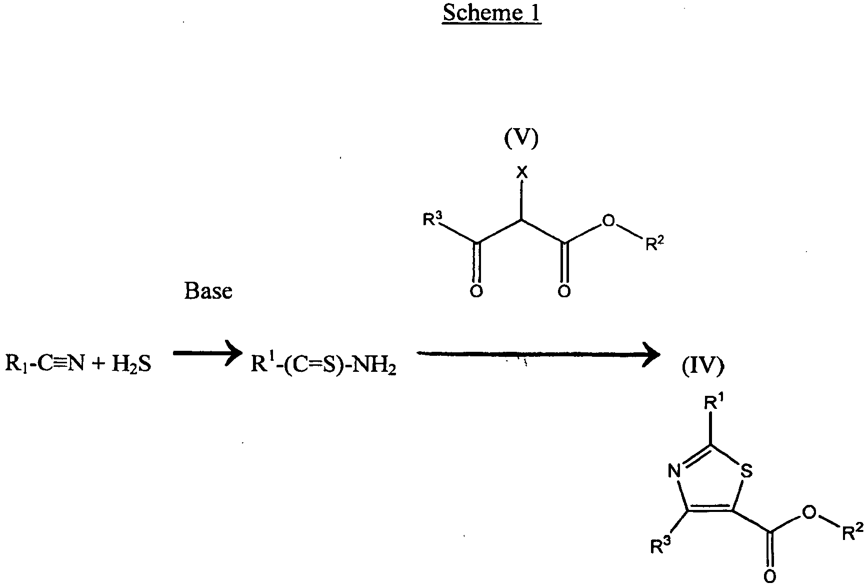

In Scheme 1, conversion of compounds of formula (II) to compounds of formula (I) can be achieved by reaction of the former with H₂S and a base in a solvent. In the invention, the solvent is ethanol. The base is a compound having formula (III)

(M)⁺(YH)⁻ (III),

in which M is sodium, potassium, lithium, or ammonium and Y is oxygen or sulfur. The reaction generally proceeds under a pressure of at least 68947.6 Pa (10 psi) and at a temperature of about 0°C to about 150°C for about 15 minutes to several days depending on the temperature and nature of the reactants. In the invention, this conversion is achieved at a pressure of 413685.4 Pa (60 psi), a temperature of 70°C, and in a solvent of ethanol.

Conversion of compounds of formula (I) to compounds of formula (IV) can be achieved By reacting compounds of formula (I) with compounds of formula (V) in a solvent. The reaction generally proceeds at a temperature of about 0°C to about 150°C for about 15 minutes to several days depending on the temperature and nature of the reactants. In the invention, this conversion is achieved at a temperature of 80°C and in a solvent of ethanol.

Ethyl 2-(4 hydroxyphenyl)-4-methyl-1,3-thiazole-5-carboxylate (a compound of formula (IV)) is prepared by reacting 4-hydroxybenzene carbothiomide (a compound of formula (I)) with ethyl-2-chloroacetoacetate (a compound of formula (V)) at a temperature of about 0°C to about 150°C in a solvent. In this invention, the temperature is 80°C and the solvent is ethanol.

Ethyl 2-(4 hydroxyphenyl)-4-methyl-1,3-thiazole-5-carboxylate (a compound of formula (IV)) can be prepared by:
(a) reacting 4-hydroxybenzonitrile (a compound of formula (II)), H2S, and sodium hydroxide (a base and compound of formula (III)) under a pressure of at least 68947.6 Pa (10 psi) at a temperature of about 0°C to about 150°C in a solvent; and
(b) reacting the product of step (a) and ethyl-2-chloroacetoacetate at a temperature of about 0°C to about 150°C in a solvent.

In this invention, in (a) the pressure is 413685.4 Pa (60 psi), the temperature is 70°C, and the solvent is ethanol. And in (b) the temperature is 80°C and the solvent is ethanol.

### Example 1 (comparative)

### 4-hydroxybenzene carbothioamide

A mixture of 4-Cyanophenol (50.0 g, 0.42mol), and NaSH (15.5g, .21mol) in distilled Water (125mL) was stirred at room temperature for 30 minutes. The mixture was then put under A vacuum, flushed with H2S, and the pressure was brought to 275790.3-344737.9 Pa (40-50 psi) for a period of a few minutes. The mixture was then heated to 70°C and stirred for 40-45 minutes. The mixture was stirred vigorously at 70°C under constant pressure of 386106.4 Pa (56 psi) for 5 hours and 15 minutes. The H2S pressure was removed and the reaction was cooled to room temperature. The reaction was neutralized to pH 5-7 with 2 M HCl (66 mL). The product was filtered, and the filter cake washed with distilled water (2x50 mL), and dried under a vacuum at 80-85°C for 22 hours to provide 62.74 g (97.57%) desired product.

### Example 2

### Ethyl 2-(4-hydroxyphenyl)-4-methyl-1,3-thiazole-5-carboxylate

A mixture of 4-Cyanophenol (23.82 g, 0.2mol), NaOH (8g, .2mol), and 200 mL ethanol were mixed in a pressure bottle while heated to 80°C. Hydrogen sulfide gas was then introduced and the pressure increased to 206842.7-413685.4 Pa (30-60 psi) until the thioamidation was determined to be complete by HPLC. Without isolating the thioamide product, HCl was added to the bottle until the pH was below 3.5, the H2S gas was removed, and the bottle was placed under a vacuum for 20 minutes at 30-40°C. The reaction was then heated to 70°C and ethyl 2-chloroacetoacetate (1.1 eq.) was added to the reaction solution. The reaction was mixed under reflux for 2-3 hours, treated with enough H₂0 to dissolve the NaCl salt in the reaction mixture, cooled to room temperature, treated with enough water to precipitate the product, and the solid was collected by filtration. The precipitate was washed with water and dried at 80°C with nitrogen bleeding to provide 50.50 g (84.2%) of desired product.

## Claims

1. A process for the preparation of a compound of formula (IV) 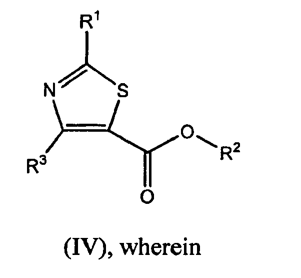
R¹ is selected from the group consisting of heteroaryl, phenyl, or phenyl substituted with one, two, three, or four substituents independently selected from the group consisting of C₁-C₆-alkyl, C₂-C₆-alkenyl, C₂-C₆-alkynyl, -OH, -F, -Cl, -Br, -I, -NH₂ and -NO₂;
R² is selected from the group consisting of hydrogen, C₁-C₆-alkyl, C₂-C₆-alkenyl, and C₂-C₆-alkynyl; and
R³ is selected from the group consisting of hydrogen, C₁-C₆-alkyl, C₂-C₆-alkenyl, and C₂-C₆-alkynyl;
the process comprising the steps of:
(a) reacting a compound having formula (II)
R¹-C≡N (II),
with a base and H₂S to provide a compound of formula (I)
R¹-(C=S)-NH₂ (I),
wherein the base is a compound of formula (III)
(M)⁺(YH)⁻ (III),
wherein
M is sodium, potassium, lithium, or -NH₄; and
Y is oxygen or sulfur;
wherein step (a) is conducted under a pressure of 413685.4 Pa (60 psi) at a temperature of 70 °C in ethanol;
and
(b) reacting the product of step (a) with a compound having formula (V) 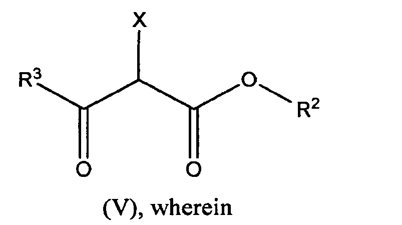
X is selected from the group consisting of -Cl, -Br, -I, and -F;
wherein step (b) is conducted at a temperature of about 80 °C in ethanol;
wherein the process is conducted as a continuous process.

2. The process according to claim 1 for the preparation of ethyl-2-(4-hydroxyphenyl)-4-methyl-1,3-thiazole-5-carboxylate.

3. The process of claim 1, wherein the base is sodium hydroxide.

## Patentansprüche

1. Ein Verfahren für die Herstellung einer Verbindung mit der Formel (IV) 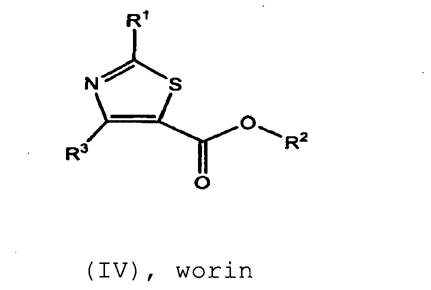
R¹ gewählt ist aus der Gruppe bestehend aus Heteroaryl, Phenyl, oder Phenyl substituiert mit einem, zwei, drei oder vier Substituenten unabhängig gewählt aus der Gruppe bestehend aus C₁-C₆-Alkyl, C₂-C₆-Alkenyl, C₂-C₆-Alkinyl, -OH, -F, -Cl, -Br, -I, -NH₂ und -NO₂;
R² ist gewählt aus der Gruppe bestehend aus Wasserstoff, C₁-C₆-Alkyl, C₂-C₆-Alkenyl und C₂-C₆-Alkinyl; und
R³ ist gewählt aus der Gruppe bestehend aus Wasserstoff, C₁-C₆-Alkyl, C₂-C₆-Alkenyl und C₂-C₆-Alkinyl;
wobei das Verfahren die folgenden Schritte umfasst:
(a) Reagieren einer Verbindung mit der Formel (II)
R¹-C≡N (II),
mit einer Base und H₂S, um eine Verbindung mit der Formel (I) zu liefern
R¹-(C=S)-NH₂ (I),
worin die Base eine Verbindung mit der Formel (III) ist
(M)⁺(YH)⁻ (III),
worin
M Natrium, Kalium, Lithium oder -NH₄ ist; und
Y ist Sauerstoff oder Schwefel;
worin Schritt (a) unter einem Druck von 413685.4 Pa (60 psi) bei einer Temperatur von 70°C in Ethanol durchgeführt wird;
und
(b) Reagieren des Produkts aus Schritt (a) mit einer Verbindung mit der Formel (V) 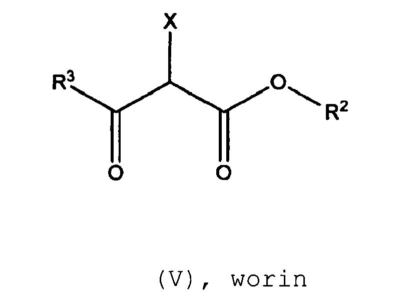
X gewählt ist aus der Gruppe bestehend aus -Cl, -Br, -I und -F; worin Schritt (b) bei einer Temperatur von ungefähr 80°C in Ethanol durchgeführt wird;
worin das Verfahren als ein kontinuierliches Verfahren durchgeführt wird.

2. Das Verfahren gemäß Anspruch 1 für die Herstellung von Ethyl-2-(4-hydroxyphenyl)-4-methyl-1,3-thiazol-5-carboxylat.

3. Das Verfahren gemäß Anspruch 1, worin die Base Natriumhydoxid ist.

## Revendications

1. Procédé pour la préparation d'un composé de formule (IV) 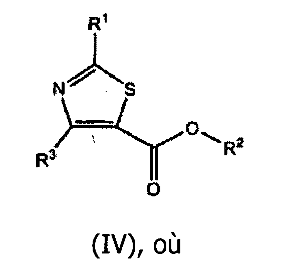
R¹ est choisi dans le groupe consistant en hétéroaryle, phényle ou phényle substitué avec un, deux, trois ou quatre substituants choisis indépendamment dans le groupe consistant en C₁-C₆-alkyle, C₂-C₆-alcényle, C₂-C₆-alcynyle, -OH, -F, -Cl, -Br, -I, -NH₂ et -NO₂ ;
R² est choisi dans le groupe consistant en l'hydrogène, C₁-C₆-alkyle, C₂-C₆-alcényle et C₂-C₆-alcynyle ; et
R³ est choisi dans le groupe consistant en l'hydrogène, C₁-C₆-alkyle, C₂-C₆-alcényle et C₂-C₆-alcynyle ;
le procédé comprenant les étapes de :
(a) réaction d'un composé ayant la formule (II)
R¹-C≡N (II),
avec une base et H₂S pour donner un composé de formule (I)
R¹-(C=S)-NH₂ (I),
où la base est un composé de formule (III)
(M)⁺(YH)⁻ (III),
où
M est le sodium, le potassium, le lithium ou -NH₄ ; et
Y est l'oxygène ou le soufre ;
où l'étape (a) est conduite sous une pression de 413685,4 Pa (60 psi) à une température de 70°C dans l'éthanol ;
et
(b) réaction du produit de l'étape (a) avec un composé ayant la formule (V) 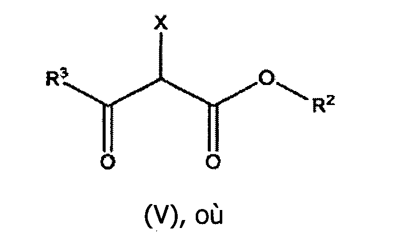
X est choisi dans le groupe consistant en -Cl, -Br, -I et -F ;
où l'étape (b) est conduite à une température d'environ 80°C dans l'éthanol ;
où le procédé est conduit comme un procédé continu.

2. Procédé selon la revendication 1 pour la préparation du 2-(4-hydroxyphényl)-4-méthyl-1,3-thiazole-5-carboxylate d'éthyle.

3. Procédé selon la revendication 1, où la base est l'hydroxyde de sodium.
